# EUROPÄISCHE PATENTSCHRIFT

(11) **EP 1 318 377 B1**
(45) Veröffentlichungstag und Bekanntmachung des Hinweises auf die Patenterteilung: **20.02.2013**
(21) Anmeldenummer: 02027485.8
(22) Anmeldetag: 10.12.2002
(51) Int. Cl.: G01B 15/02

(54) **Verfahren und Vorrichtung zur Prüfung einer Matte aus Biomassepartikeln**
Method and device for testing a mat made of biomass particles
Méthode et dispositif pour évaluer une matte composée de particules de biomasse

(30) Priorität: 10.12.2001 DE 10160398
(43) Veröffentlichungstag der Anmeldung: 11.06.2003
(73) Patentinhaber: Dieffenbacher GmbH Maschinen- und Anlagenbau, 75031 Eppingen (DE)
(72) Erfinder: Dueholm, Sten, 2900 Hellerup (DK)
(74) Vertreter: Hartdegen, Anton

(56) Entgegenhaltungen:
- CA-A- 1 202 431
- DE-A- 3 429 135
- GB-A- 2 054 841
- US-A- 4 047 036
- ADAMS D W ET AL: "HIGH RESOLUTION SOLID STATE SENSOR FOR STRIP EDGE DROP AND THICKNESS PROFILE" IRON AND STEEL ENGINEER, ASSOCIATION OF IRON AND STEEL ENGINEERS. PITTSBURGH, US, Bd. 75, Nr. 9, 1. September 1998 (1998-09-01), Seiten 33-36, XP000788068 ISSN: 0021-1559
- S.GONDROM E.A.: "Digital computed laminography and tomosynthesis-functional principles and industrial applications" NDT.NET, [Online] Bd. 4, Nr. 7, Juli 1999 (1999-07), XP008014911 Gefunden im Internet: <URL:NDT.net> [gefunden am 2003-03-13]
- PATENT ABSTRACTS OF JAPAN vol. 010, no. 097 (P-446), 15. April 1986 (1986-04-15) & JP 60 230009 A (TOSHIBA KK), 15. November 1985 (1985-11-15)
- PATENT ABSTRACTS OF JAPAN vol. 010, no. 097 (P-446), 15. April 1986 (1986-04-15) & JP 60 230010 A (TOSHIBA KK), 15. November 1985 (1985-11-15)

## Beschreibung

Die Erfindung betrifft ein Verfahren nach dem Oberbegriff des Anspruchs 1 und eine Vorrichtung nach dem Oberbegriff des Anspruchs 11.

Die zu prüfenden Matten bestehen insbesondere aus Fasern und/oder Holzspänen und werden letztlich vorzugsweise in einer kontinuierlich arbeitenden Doppelbandpresse zu Spanplatten, Faserplatten, OSB-Platten und ähnlichen Platten verpresst. Die heute üblichen, kontinuierlich arbeitenden Doppelbandpressen arbeiten isochor, d.h., mit vorgegebener Distanz zwischen den Pressplatten. Diese Doppelbandpressen weisen gegenläufige, die Matte auf eine Enddicke komprimierende Stahlbänder auf. Fremdkörper und Bereiche der Matte, die sich nicht auf diese Enddicke komprimieren lassen, können zu Beulen, Rissen oder gar Ausstanzungen in den Stahlbändern und sogar zu Beschädigungen der die Stahlbänder stützenden Rollenstab- bzw. Rollenkettensysteme und Heizplatten führen.

### Stand der Technik

Zur Vermeidung solcher Schäden ist es bekannt, in der Plattenfertigung Metallsuchgeräte einzusetzen, die magnetisierbare und nicht magnetisierbare Metallgegenstände in der Matte detektieren. Wenn solche Metallgegenstände festgestellt werden, wird das quer zu seiner Bewegungsrichtung geteilte Formband in der Bewegungsrichtung auseinandergefahren, und der fehlerhafte Mattenteil wird in einen Abwurfschacht abgeführt. Nach dem Schließen des Formbandes wird der Fertigungsprozess mit einer fehlerfreien Matte fortgesetzt. Magnetisierbare Metallgegenstände werden auch durch Magnete aus der Matte herausgezogen.

Bei einer bekannten Vorrichtung der eingangs genannten Art (vgl. US 4,047,036 A = DE 26 18 906 A1) sind auf einer Seite eines Bandes zur Ermittlung dessen Dicke zwei Strahlungsquellen angeordnet, deren Ausgangsstrahlung zu je einem Strahlungsfächer geformt werden, welche zueinander ausgefluchtet die Breite des Bandes überlagern und eine Zone auf der gegenüberliegenden Seite des Bandes bestrahlen, welche eine auf den Empfang von Strahlung ausgebildete Detektorvorrichtung aufweist.

Vergleichbares gilt für die aus der JP 60230009 A und JP 60230010 A bekannten Vorrichtungen, bei welchen auf einer Seite eines plattenförmigen Produkts in ebenfalls nur einer Reihe quer zum Produkt nunmehr drei Strahlungsquellen angeordnet sind, deren Ausgangsstrahlung zu je einem Strahlungsfächer geformt wird. Ebenfalls in nur einer Reihe sind auf der anderen Seite des Produkts drei Anordnungen von Detektoren vorgesehen, wobei jede Anordnung einen Kreisbogen bildet, dessen Mittelpunkt einer der drei Strahlungsquellen zugeordnet ist.

Aus der CA 1 202 431 A ist es an sich bekannt, auf einer Seite eines plattenförmigen Produkts nur eine Strahlungsquelle anzuordnen, deren Ausgangsstrahlung zu einem Strahlungsfächer geformt wird. Die Breitenerstreckung ist quer zu der Bewegungsrichtung des Produkts angeordnet. Die Detektoren sind auf der anderen Seite des Produkts in nur einer Reihe auf einem Kreisbogen angeordnet, dessen Mittelpunkt die Strahlungsquelle ist. Die Detektoren sind auf den Strahlungsfächer ausgerichtet. Ein ganz außen positionierter Detektor empfängt Strahlung der Strahlungsquelle, die zuvor nicht durch das Produkt hindurchgedrungen ist. Dieser äußerste Detektor dient zur selbsttätigen Kalibrierung der bekannten Vorrichtung. Mit dieser Vorrichtung soll die Masse je Flächeneinheit des Produkts ermittelt werden. Bei konstanter Dichte des Produkts kann aus der Masse je Flächeneinheit die Dicke des Produkts ermittelt werden. Andererseits lässt sich bei konstanter Dicke des Produkts aus der Masse je Flächeneinheit auf die Dichte des Produkts schließen.

Schließlich sei noch auf die DE 34 29 135 A1 hingewiesen.

### Aufgabenstellung

Der Erfindung liegt die Aufgabe zugrunde, außer den metallischen Gegenständen auch andere Fremdkörper unerwünscht großer Dichte in der Matte festzustellen und zu verhindern, dass diese Fremdkörper in die nachgeschaltete Doppelbandpresse gelangen und dort zu örtlichen Konzentrationen zu großer Dichte führen.

Diese Aufgabe ist hinsichtlich des Verfahrens durch die Merkmale des Anspruchs 1 gelöst. Als Strahlungsquellen sind insbesondere Röntgenstrahler geeignet. Die Matte kann auf diese Weise ganzflächig geprüft werden. Jedes Detektorelement kann mehrere Detektorzellen aufweisen. Dabei ergeben sich in Abhängigkeit von der Anzahl der Detektorzellen mit beliebig hoher Auflösung Informationen über die Dichte der Matte. Es kann jeweils die optimale Anzahl Strahlungsquellen verwendet werden. Vorzugsweise ist der Abstand der Strahlungsquellen voneinander einstellbar. Die Strahlungsfächer führen zu einer kompakten Bauweise und zu hoher Betriebssicherheit. Die Überlappungen dienen einerseits der sicheren Datenerfassung über die gesamte Breite der Matte und andererseits der Verbesserung der Auswertung der erfassten Daten. Durch den Längsabstand ist sichergestellt, dass von jeder Strahlungsquelle Strahlung nur auf die zugehörige Zeile von Detektorelementen gelangt.

Gemäß Anspruch 2 oder 3 lassen sich Werte rechtwinklig zur Oberfläche der Matte errechnen.

Gemäß Anspruch 4 können alle im praktischen Betrieb auftretenden Fremdkörper festgestellt und mit dem zugehörigen Abschnitt der Matte entfernt werden. Trotz größter Sorgfalt bei der Prozessführung gelangen immer wieder Fremdkörper unterschiedlicher Art und Größe in die Matte. Hierzu gehören Metallteile aus dem Rohholz oder von vorgeschalteten Prozessstufen, Metall- und Kunststoffteile aus eventuellen Abfallbeigaben, verfestigte oder ausgehärtete Leimklumpen aus der Bindemittelaufbringvorrichtung oder überdichte Partikelaggregate, die sich in verschiedenen Stadien des Prozesses bilden können. Diese Fremdkörper liegen als unsichtbare, überdichte Stellen in der Matte vor. Da Holz nicht über seine Rohdichte von etwa 1500 kg/m³ hinaus verdichtet werden kann, können auch diese überdichten Stellen in der Matte nicht auf die an der Heißpresse eingestellte Enddicke der fertigen Platte zusammengedrückt und noch weiter verdichtet werden. Um Schaden von der Heißpresse fernzuhalten, bringt es daher erhebliche Vorteile, gemäß Anspruch 4 sämtliche Fremdkörper aus der Matte entfernen zu können. Die Vorrichtung zur Entfernung eines Fremdkörper enthaltenden Abschnitts der Matte kann in an sich bekannter Weise ein quer zu seiner Bewegungsrichtung geteiltes Formband aufweisen, das zur vorübergehenden Bildung eines Schlitzes auseinandergefahren werden kann. Dem Schlitz ist ein Abwurfschacht nachgeschaltet, in den der fehlerhafte Mattenabschnitt abgeworfen wird. Anschließend wird der Schlitz des Formbandes wieder geschlossen und der Prozess fortgesetzt.

Gemäß Anspruch 5 lässt sich mit den gewonnenen Dichtedaten auch die Masse je Flächeneinheit der Matte flächendeckend ermitteln. Dadurch besteht die Möglichkeit, die Matte auch hinsichtlich unerwünschter, von der Streumaschine verursachter Variationen der Masse je Flächeneinheit zu überwachen und bei Bedarf Korrekturmaßnahmen einzuleiten.

Gemäß Anspruch 6 ist eine einfache und sichere Kalibrierung der Einheiten aus Strahlungsquelle und zugeordneten Detektorelementen ermöglicht. Vorzugsweise wird nur an einem der beiden Längsränder der Matte ein Normkörper angeordnet.

Die Ansprüche 7 und 8 kennzeichnen jeweils eine günstige Vorgehensweise für die Kalibrierung.

Der Erfindung liegt auch die Aufgabe zugrunde, die vorgeschaltete Streumaschine zu regeln.

Diese Aufgabe ist durch die Merkmale des Anspruchs 9 gelöst. Die von der Matte flächendeckend gewonnenen Dichtedaten lassen sich in vorteilhafter Weise auch zur Regelung der vorgeschalteten Streumaschine verwenden. Jeder Längsstreifen kann z.B. eine Breite von 10 bis 20 cm aufweisen. Die Breite der Längsstreifen kann auf die Breite der Reaktionsmechanismen in der Streumaschine eingestellt werden. Es sind verschiedene derartige Reaktionsmechanismen an sich bekannt, die es gestatten, den zugehörigen Längsstreifen der Matte dichter oder weniger dicht zu streuen.

Gemäß Anspruch 10 lassen sich Mattenabschnitte zu geringer Dichte entfernen.

Die eingangs erwähnte Aufgabe ist hinsichtlich der Vorrichtung durch die Merkmale des Anspruchs 11 gelöst. Hier ergeben sich die gleichen Vorteile wie zu Anspruch 1 erläutert.

Gemäß Anspruch 12 kann in Abhängigkeit von der Art der zu prüfenden Matte die Anzahl der Strahlungsquellen und die Auswertung der gewonnenen elektrischen Ausgangssignale optimiert werden.

Die Merkmale des Anspruchs 13 bieten betriebliche und kostenmäßige Vorteile, ebenso wie die Merkmale der Ansprüche 14 und 15.

### Ausführungsbeispiel

Diese und weitere Merkmale und Vorteile der Erfindung werden nachfolgend anhand des in den Zeichnungen dargestellten Ausführungsbeispiels näher erläutert. Es zeigt
- Fig. 1: schematisch ein Flussdiagramm für die Herstellung der Matten und Platten,
- Fig. 2: das Detail II gemäß Fig. 1 in vergrößerter Darstellung,
- Fig. 3: eine Seitenansicht einer Prüfvorrichtung,
- Fig. 4: die Schnittansicht nach Linie IV-IV in Fig. 3 in vergrößerter Darstellung und
- Fig. 5: schematisch die Ansicht gemäß V-V in Fig. 3 mit zugehöriger Schaltungsanordnung.

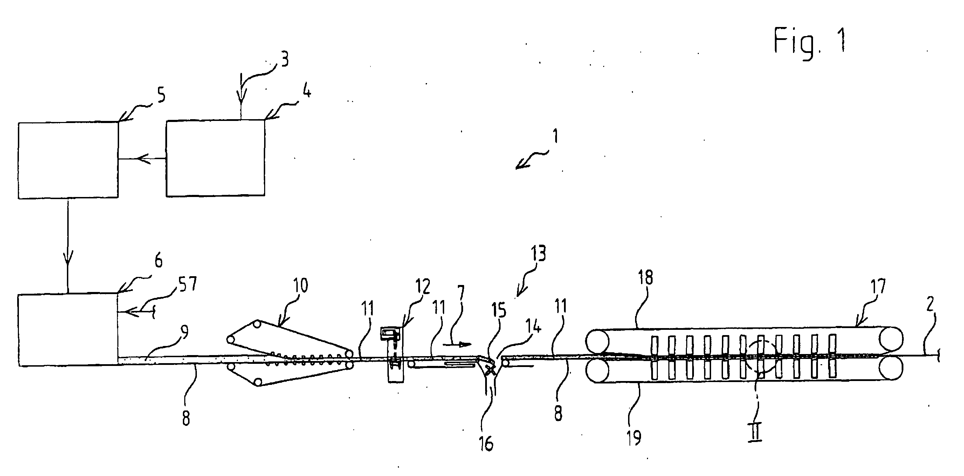

Fig. 1 zeigt schematisch eine Anlage 1 zur kontinuierlichen Herstellung von Platten 2 aus Biomassepartikeln, die gemäß einem Pfeil 3 in einen Bunker 4 eingebracht werden. Aus dem Bunker 4 gelangen die Biomassepartikel, insbesondere Fasern und/oder Holzspäne, in eine Bindemittelaufbringvorrichtung 5, aus der sie in eine Streumaschine 6 eingebracht werden.

Die Streumaschine 6 streut die Biomassepartikel in an sich bekannter Weise auf ein sich in einer Bewegungsrichtung 7 bewegendes Formband 8. Auf dem Formband 8 entsteht auf diese Weise ein Vlies oder eine Matte 9 aus den Biomassepartikeln.

Vorzugsweise in einer kontinuierlichen Kaltpresse 10 wird die Matte 9 sodann vorkomprimiert. Die vorkomprimierte Matte 11 durchläuft anschließend eine Prüfvorrichtung 12. In der Prüfvorrichtung 12 wird einerseits die vorkomprimierte Matte 11 auf Fremdkörper 26, wie Metallteile, Leimklumpen, Kunststoffteile, überdichte Partikelaggregate und ähnliche Teile, untersucht. Andererseits kann durch die Prüfvorrichtung 12 bei Bedarf zusätzlich die Masse je Flächeneinheit der Matte 11 flächendeckend festgestellt werden.

Das Formband 8 ist im Bereich einer Vorrichtung 13 zur Entfernung eines Fremdkörper 26 enthaltenden Abschnitts 15 der vorkomprimierten Matte 11 quer zu der Bewegungsrichtung 7 geteilt und lässt sich, wenn durch die Prüfvorrichtung 12 ein Fehler in der vorkomprimierten Matte 11 festgestellt worden ist, unter Bildung eines Spalts 14 auseinanderfahren. Dadurch kann ein den Fehler enthaltender Abschnitt 15 der vorkomprimierten Matte 11 in einen Abwurfschacht 16 abgeworfen werden. Sobald dies geschehen ist, wird das Formband 8 wieder zusammengefahren und der Spalt 14 geschlossen. Die vorkomprimierte Matte 11 läuft sodann in der Bewegungsrichtung 7 weiter in eine kontinuierlich arbeitende Heißpresse 17. In der Heißpresse 17 wird die vorkomprimierte und von den erwähnten Fehlern befreite Matte 11 unter Einwirkung von Druck und Wärme zu der fertigen Platte 2 verpresst. Dabei härtet das in der Bindemittelaufbringvorrichtung 5 auf die Biomassepartikel aufgebrachte thermohärtende Bindemittel aus und führt zur Bindung der Partikel aneinander und zur Verfestigung der fertigen Platte 2.

Vorzugsweise handelt sich bei der Heißpresse 17 um eine an sich bekannte Doppelbandpresse, in der die Platte 2 zwischen einem oberen Pressband 18 und einem unteren Pressband 19 verpresst wird. Die Pressbänder 18, 19 bestehen aus über die gesamte Breite der Platte 2 reichenden Stahlbändern von z.B. 2,5 mm Dicke.

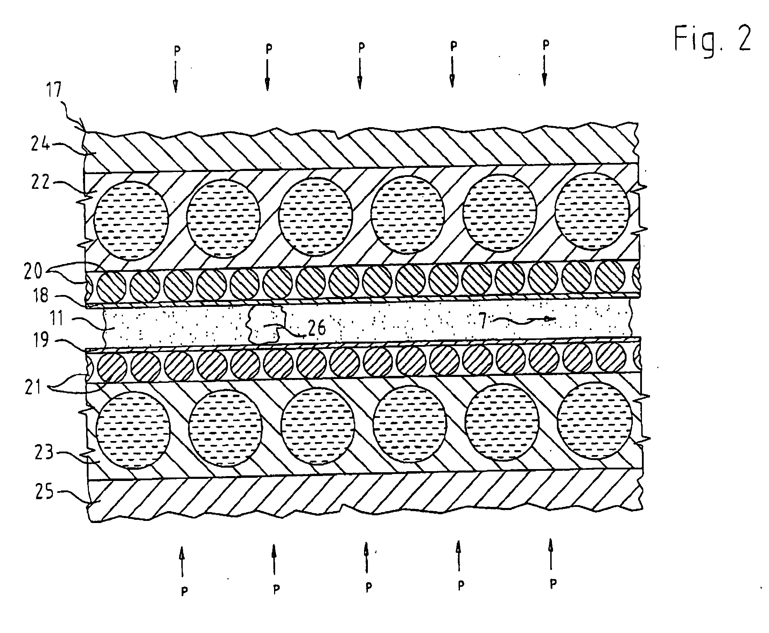

Weitere Einzelheiten der Heißpresse 17 sind in Fig. 2 dargestellt. Die Pressbänder 18, 19 liegen an oberen Rollenstäben 20 und unteren Rollenstäben 21 an, die ihrerseits an einer oberen Heizplatte 22 oder einer unteren Heizplatte 23 abgestützt sind. Die obere Heizplatte 22 liegt an einer Pressplatte 24 an, während die untere Heizplatte 23 auf einem Presstisch 25 aufliegt. Presskräfte P werden in an sich bekannter Weise auf das System aufgebracht.

Wenn die vorkomprimierte Matte 11 gemäß Fig. 2 einen oder mehrere Fremdkörper 26 unerwünscht großer Dichte enthält, die sich nicht über die Rohdichte von Holz von etwa 1500 kg/m³ hinaus verdichten lassen, können diese Fremdkörper 26 nicht auf die an der Heißpresse 17 eingestellte Enddicke der fertigen Platte 2 (Fig. 1) zusammengedrückt und noch weiter verdichtet werden. Gleiches gilt selbstverständlich für metallische Fremdkörper 26. Außer metallischen Fremdkörpern 26 können Fremdkörper 26 in Form von Leimklumpen, die sich aus dem Bindemittelaufbringsystem lockern, in die Matte 11 geraten. Es treten auch Fremdkörper in Form von Metall- und Kunststoffteilen aus Abfallbeimengungen auf. Als Fremdkörper 26 kommen ferner Faserknollen hoher Dichte mit hohem Beleimungsanteil vor, die sich manchmal in der Streumaschine entwickeln. Solche Fremdkörper 26 können unterschiedliche Größe haben. Im MDF-Bereich ist mit Fremdkörpern 26 einer Größe von 2 bis 3 mm zu rechnen, im OSB-Bereich mit viel größeren Fremdkörpern 26 bis zu 5 cm Größe.

Da bekannte Heißpressen 17 isochor, d.h. mit vorgegebenem Abstand zwischen der Pressplatte 24 und dem Presstisch 25, arbeiten, können die Pressbänder 18, 19 vor den Fremdkörpern 26 nicht ausweichen und werden sehr leicht durch die Fremdkörper 26 beschädigt. Diese Beschädigungen können sich in Beulen, Rissen oder gar Perforationen der Pressbänder äußern. Schlimmstenfalls können auch die Rollenstäbe 20, 21 und die Heizplatten 22, 23 Schaden nehmen. Es ist also von besonderer Bedeutung und wesentliches Anliegen der Erfindung, dafür zu sorgen, dass sich beim Einlauf der vorkomprimierten Matte 11 in die Heißpresse 17 keine unerwünschten Fremdkörper 26 mehr in der vorkomprimierten Matte 11 befinden.

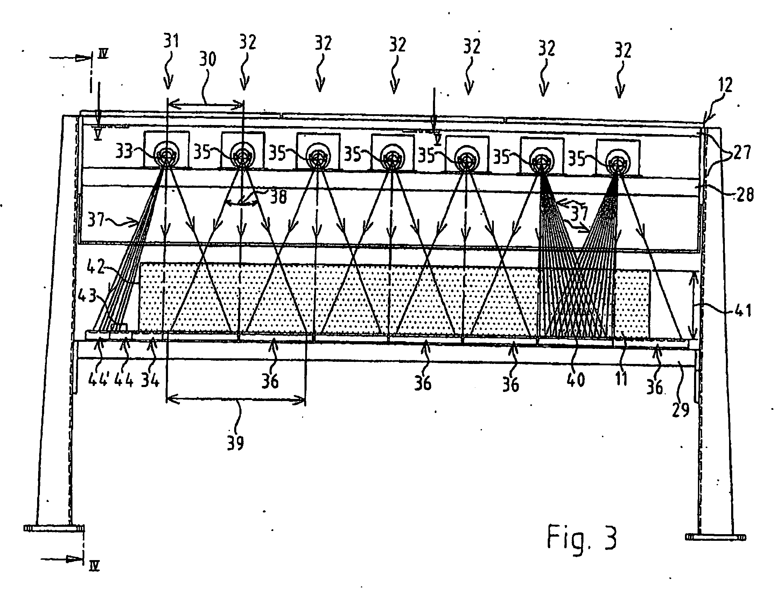

Fig. 3 zeigt Einzelheiten der Prüfvorrichtung 12. Diese weist einen Rahmen 27 mit einer oberen Traverse 28 und einer unteren Traverse 29 auf.

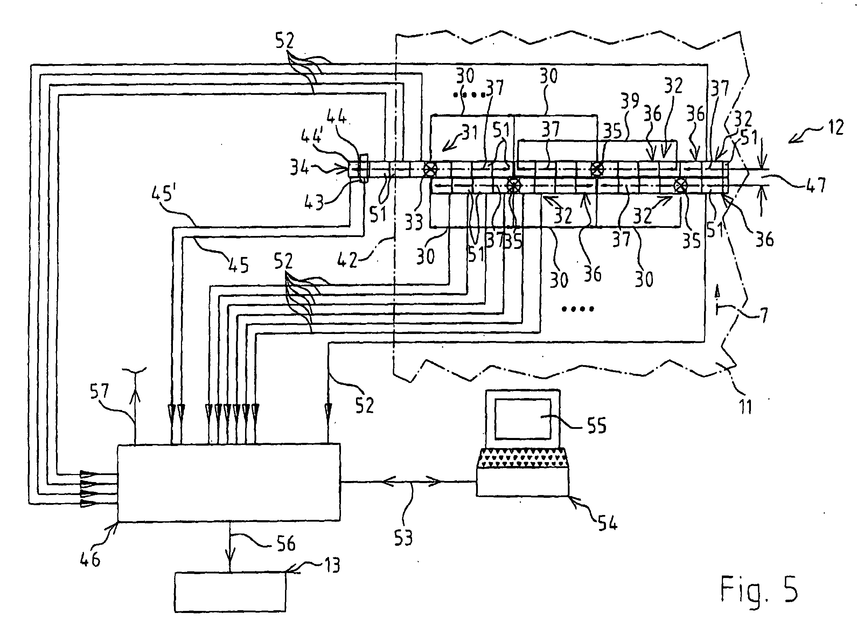

Die Bewegungsrichtung 7 (Fig. 1) verläuft in Fig. 3 senkrecht zur Zeichenebene. Quer zu dieser Bewegungsrichtung 7 sind an den Traversen 28, 29 eine äußere Einheit 31 und sonstige Einheiten 32 montiert. Die äußere Einheit 31 weist eine äußere Strahlungsquelle 33 und eine sich quer zu der Bewegungsrichtung 7 erstreckende äußere Zeile 34 von Detektorelementen 44, 44', 51 ... (Fig. 5) auf, die auf der unteren Traverse 29 montiert sind. Jede sonstige Einheit 32 besteht aus einer Strahlungsquelle 35 an der oberen Traverse 28 und einer sich quer zu der Bewegungsrichtung 7 erstreckenden sonstigen Zeile 36 von Detektorelementen 51 ... (Fig. 5), die wiederum an der unteren Traverse 29 montiert ist. Jedes Detektorelement 44, 44', 51 weist eine Zeile von z.B. 128 (nicht gezeichneten) Detektorzellen (Pixel) auf. Aus den Detektorzellen können die Ausgangssignale z.B. periodisch als Datenstrang von Dichtewerten abgerufen und ausgewertet werden.

Die von jeder Strahlungsquelle 33, 35 ausgehende Strahlung ist zu einem Strahlungsfächer 37 mit einem Öffnungswinkel 38 geformt, der 30° bis 60°, vorzugsweise 44°, beträgt. Eine Breitenerstreckung 39 jedes Strahlungsfächers 37 ist quer zu der Bewegungsrichtung 7 der vorkomprimierten Matte 11 angeordnet und fluchtet mit der zugehörigen Zeile 34, 36 von Detektorelementen 44, 44', 51 ... ; 51 ... . Gemäß Fig. 3 sind distale Enden benachbarter Strahlungsfächer 37 quer zu der Bewegungsrichtung 7 überlappend angeordnet. Dies ist für die beiden sonstigen Einheiten 32 rechts in Fig. 3 besonders deutlich dargestellt. Für den dreieckigen Überlappungsbereich 40 benachbarter Strahlungsfächer 37 erhält man bei Durchstrahlung der vorkomprimierten Matte 11 eine Doppelinformation zur Dichte der vorkomprimierten Matte 11. Diese Doppelinformation kann in später zu beschreibender Weise bei der rechnerischen Ermittlung der Masse je Flächeneinheit der vorkomprimierten Matte 11 herangezogen werden. So ist es vorteilhaft, wenn die Höhe des Überlappungsbereichs 40 mindestens gleich einer Dicke 41 der vorkomprimierten Matte 11 ist. Dadurch kann die Dichteinformation über die gesamte Breite der Matte gewonnen werden.

Wie Fig. 3 zeigt, geht ein linker Teil des Strahlungsfächers 37 der äußeren Einheit 31 an einem Längsrand 42 der vorkomprimierten Matte 11 vorbei und durchdringt einen auf der äußeren Zeile 34 von Detektorelementen 44, 44', 51 ... liegenden Normkörper 43 mit bekannter Masse je Flächeneinheit. Dieser Strahlungsanteil, der nur den Normkörper 43 durchdrungen hat, jedoch nicht die vorkomprimierte Matte 11, wird von wenigstens einem äußeren Detektorelement 44 (s. auch Fig. 5) der äußeren Zeile 34 von Detektorelementen 44, 44', 51 ... aufgenommen und in elektrische Ausgangssignale gewandelt. Diese Ausgangssignale gelangen gemäß Fig. 5 über eine Leitung 45 in eine Auswerteschaltung 46 und werden dort zur Kalibrierung der äußeren Einheit 31 und der sonstigen Einheiten 32 verwendet. Ein äußerer Teil des linken Strahlungsfächers 37 in Fig. 3 geht an dem Normkörper 43 vorbei und wird von wenigstens einem äußeren Detektorelement 44' der äußeren Reihe 34 von Detektorelementen 44, 44', 51 ... aufgenommen. Das äußere Detektorelement 44' kann in Fig. 3 links (wie gezeichnet) oder rechts von dem äußeren Detektorelement 44 angeordnet sein. Der durch das äußere Detektorelement 44' aufgenommene Strahlungsanteil wird in Referenzausgangssignale gewandelt, die über eine Leitung 45' (Fig. 5) der Auswerteschaltung 46 zugeführt und zur Kalibrierung der äußeren Einheit 31 verwendet werden. Alle sonstigen Einheiten 32 können mit der äußeren Einheit 31 abgeglichen werden. Es kann aber auch zunächst die der äußeren Einheit 31 benachbarte sonstige Einheit 32 entsprechend der äußeren Einheit 31 kalibriert werden, worauf alle übrigen sonstigen Einheiten 32 nacheinander entsprechend kalibriert werden.

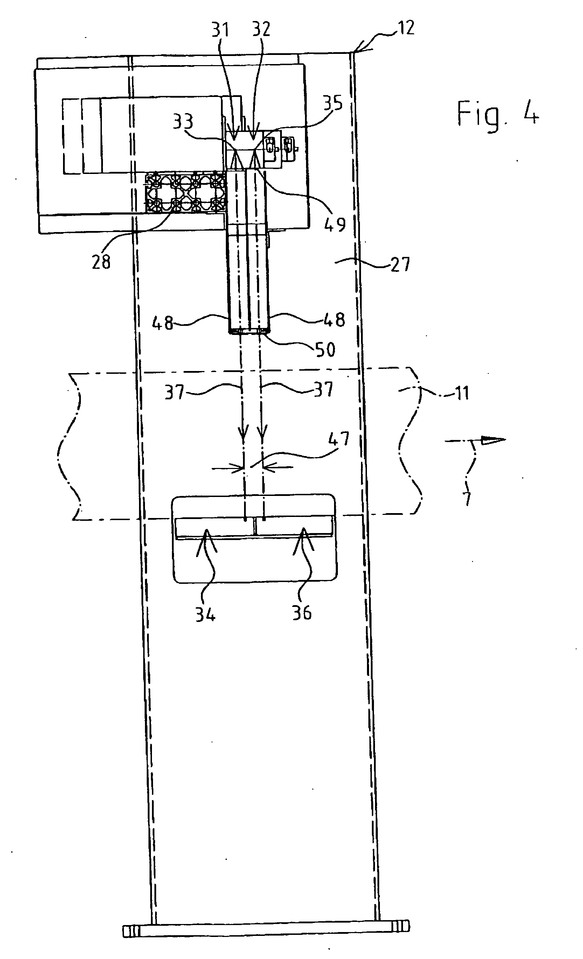

Gemäß Fig. 4 sind benachbarte Strahlungsfächer 37, 37 und die jeweils zugehörigen Zeilen 34, 36 der Detektorelemente 44, 44', 51 ...; 51 ... in der Bewegungsrichtung 7 der vorkomprimierten Matte 11 in einem Längsabstand 47 von z.B. 50 mm voneinander angeordnet.

Als Strahlungsquellen 33, 35 werden vorzugsweise konventionelle Röntgenröhren eingesetzt, die im Prinzip wie Punktstrahler wirken. Die Strahlungsfächer 37 werden durch Kollimatorschächte 48 geformt, wobei jeder Kollimatorschacht 48 einen oberen Kollimatorschlitz 49 und einen unteren Kollimatorschlitz 50 aufweist.

Wie Fig. 5 schematisch zeigt, weist die äußere Zeile 34 von Detektorelementen außer den äußeren Detektorelementen 44, 44' noch Detektorelemente 51 ... auf. Die sonstigen Zeilen 36 von Detektorelementen bestehen ebenfalls aus solchen Detektorelementen 51 ... . Jedes Detektorelement 51 ist über eine Verbindungsleitung 52 mit der Auswerteschaltung 46 verbunden. Zur Vereinfachung sind in Fig. 5 nur einige dieser Detektorelemente 51 und ihrer Verbindungsleitungen 52 gezeichnet. Fig. 5 verdeutlicht auch, wie der Strahlungsfächer 37 jeder Einheit 31, 32 mit seiner Zeile 34, 36 von Detektorelementen 44, 44', 51 ... ; 51 ... fluchtend ausgerichtet ist. So gelangen die elektrischen Ausgangssignale aller Detektorelemente 44, 44', 51 in die Auswerteschaltung 46, wo sie verarbeitet werden. Über eine Leitung 53 ist die Auswerteschaltung 46 mit einer Eingabe/Ausgabeeinheit 54 mit Monitor 55 verbunden.

Werden durch die Prüfvorrichtung 12 Fremdkörper 26 (Fig. 2) in der vorkomprimierten Matte 11 festgestellt, steuert die Auswerteschaltung 46 über eine Leitung 56 die Vorrichtung 13 zur Entfernung des die Fremdkörper 26 enthaltenden Abschnitts 15 der Matte 11.

Die Detektorelemente 51 liefern elektrische Ausgangssignale, die der Dichte der durchstrahlten vorkomprimierten Matte 11 proportional sind. Bei der gewählten linearen Anordnung der Zeilen 34, 36 von Detektorelementen 44, 44', 51 ...; 51 ... entstehen über die Länge jeder Zeile 34, 36 variierende Entfernungen von der zugehörigen Strahlungsquelle 33, 35 bis zu den Detektorelementen 51. Für die einzelnen Strahlen jedes Strahlungsfächers 37 bestehen ferner unterschiedlich lange Strahlungswege in der vorkomprimierten Matte 11 und unterschiedlich angestrahlte Flächen der zugehörigen Detektorelemente 51. Die Wirkungen dieser variierenden geometrischen Verhältnisse lassen sich jedoch durch einfache trigonometrische Umrechnungen unter Berücksichtigung des Winkels zwischen dem jeweiligen Strahl des Strahlungsfächers 37 und der Senkrechten kompensieren.

Für das Auffinden von Fremdkörpern 26 in der vorkomprimierten Matte 11 sind diese Korrekturberechnungen ausreichend, da nur das Vorliegen und die Masse der Fremdkörper 26 entscheidend sind, nicht dagegen ihre exakte Lokalisierung.

Bei der Bestimmung der Masse je Flächeneinheit der vorkomprimierten Matte 11 ist dagegen eine weitere Datenverarbeitung in der Auswerteschaltung 46 erforderlich. Die Masse je Flächeneinheit der vorkomprimierten Matte 11 soll für senkrecht verlaufende Abschnitte der vorkomprimierten Matte 11 angegeben werden. Die aufgrund des fächerförmigen Strahlungsganges unterschiedlich orientierten Messungen sind daher auf entsprechende Ergebnisse aus senkrechten Schnitten umzurechnen. Die Umrechnung stützt sich auf die zweifachen Messungen aus verschiedenen Richtungen in dem dreieckigen Überlappungsbereich 40 (Fig. 3) benachbarter Einheiten 31, 32; 32, 32; 32, 32 .... Geeignete Rechenmodelle lehnen sich an die an sich bekannte Technik der digitalen Laminographie und Tomosynthese an, vgl. den Aufsatz von S. Gondrom und S. Schröpfer, FhG ITFP, Saarbrücken, Deutschland, mit dem Titel "Digital computed laminography and tomosynthesis - functional principles and industrial applications", veröffentlicht in NDT.net - ,Juli 1999, Vol. 4 No. 7.

Vorzugsweise werden die Ausgangssignale der Detektorelemente 51 über die Breite der vorkomprimierten Matte 11 in der Auswerteschaltung 46 zu aufeinanderfolgenden Gruppen von Ausgangssignalen gruppiert. Die jeweils die Dichte eines zu der Bewegungsrichtung 7 parallelen Längsstreifens der vorkomprimierten Matte 11 repräsentierenden elektrischen Ausgangssignale der Detektorelemente 51 jeder Gruppe werden in der Auswerteschaltung 46 zusammengefasst verarbeitet. Jede derart verarbeitete Ausgangssignalgruppe führt zu einer Regelgröße, die über eine Leitung 57 (s. auch Fig. 1) zur Regelung eines dem betreffenden Längsstreifen der vorkomprimierten Matte 11 zugeordneten Reaktionsmechanismus der Streumaschine 6 verwendet wird.

### Bezugszeichenliste

- 1: Anlage zur kontinuierlichen Herstellung von Platten 2 aus Biomassepartikeln
- 2: fertig verfestigte Platten aus Biomasseartikeln
- 3: Pfeil zur Kennzeichnung des Einbringens der Biomassepartikel in einen Bunker 4
- 4: Bunker
- 5: Bindemittelaufbringvorrichtung
- 6: Streumaschine
- 7: Bewegungsrichtung
- 8: Formband
- 9: Vlies oder Matte aus gestreuten Biomassepartikeln
- 10: Kaltpresse
- 11: vorkomprimierte Matte
- 12: Prüfvorrichtung
- 13: Vorrichtung zur Entfernung eines Fremdkörper 26 enthaltenden Abschnitts 15 der vorkomprimierten Matte 11
- 14: Spalt
- 15: Fehler enthaltender Abschnitt der vorkomprimierten Matte 11
- 16: Abwurfschacht
- 17: Heißpresse
- 18: oberes Pressband
- 19: unteres Pressband
- 20: obere Rollenstäbe
- 21: untere Rollenstäbe
- 22: obere Heizplatte
- 23: untere Heizplatte
- 24: Pressplatte
- 25: Presstisch
- P: Presskräfte
- 26: Fremdkörper, wie Metallteile, Leimklumpen, Kunststoffteile, überdichte Partikelaggregate und ähnliche Teile
- 27: Rahmen der Prüfvorrichtung 12
- 28: obere Traverse der Prüfvorrichtung 12
- 29: untere Traverse der Prüfvorrichtung 12
- 30: Querabstand zwischen zwei Strahlungsquellen 33, 35, 35 ...
- 31: äußere Einheit, bestehend aus einer äußeren Strahlungsquelle 33 und einer äußeren Zeile 34 von Detektorelementen 44, 44', 51 ...
- 32: sonstige Einheiten, bestehend aus einer Strahlungsquelle 35 und einer Zeile 36 von Detektorelementen 51 ...
- 33: äußere Strahlungsquelle der äußeren Einheit 31, vorzugsweise Röntgenröhren
- 34: äußere Zeile der Anordnung von Zeilen 34, 36 von Detektorelementen 44, 44', 51 ...
- 35: Strahlungsquelle einer sonstigen Einheit 32, vorzugsweise Röntgenröhren
- 36: sonstige Zeile der Anordnung von Zeilen 34, 36 von Detektorelementen 51 ...
- 37: Strahlungsfächer der Strahlenquellen 33, 35
- 38: Öffnungswinkel 38 der Strahlungsfächer 37
- 39: Breitenerstreckung jedes Strahlungsfächers 37
- 40: dreieckiger Überlappungsbereich benachbarter Einheiten 31, 32; 32, 32; 32, 32 ...
- 41: Dicke der vorkomprimierten Matte 11
- 42: Längsrand der vorkomprimierten Matte 11
- 43: auf der äußeren Zeile 34 von Detektorelementen 44, 44', 51 liegender Normkörper
- 44,44': äußere Detektorelemente der äußeren Zeile 34
- 45,45': Leitungen zur Auswertschaltung 46
- 46: Auswerteschaltung
- 47: Längsabstand zwischen benachbarten Strahlungsfächern 37, 37 einerseits und zwischen den jeweils zugehörigen Zeilen 34, 36 von Detektorelementen 44, 44', 51 ...; 51 ... andererseits
- 48: Kollimatorschacht
- 49: oberer Kollimatorschlitz
- 50: unterer Kollimatorschlitz
- 51: Detektorelement
- 52: Verbindungsleitung zur Auswerteschaltung 46
- 53: Leitung
- 54: Eingabe/Ausgabeeinheit
- 55: Monitor
- 56: Leitung
- 57: Leitung zur Übermittlung einer Regelgröße an die Streumaschine

## Patentansprüche

1. Verfahren zur Prüfung einer sich in einer Richtung (7) bewegenden Matte (11) aus Biomassepartikeln, insbesondere aus Fasern und/oder Holzspänen, zur Herstellung von Platten (2),
bei dem auf einer ersten Seite der Matte (11) eine sich quer zu der Bewegungsrichtung (7) der Matte (11) erstreckende Anordnung von Zeilen (34, 36) von Detektorelementen (44, 44', 51 ... ; 51 ...) vorgesehen wird,
- wobei die Detektorelemente (44, 44', 51) in einer zu der Matte (11) parallelen Ebene angeordnet werden,
- wobei die Detektorelemente (51) Strahlung aufnehmen, die von einer der ersten Seite gegenüberliegenden zweiten Seite der Matte (11) ausgegangen ist und die Matte (11) durchdrungen hat,
- und wobei durch jedes Detektorelement (44, 44', 51) der aufgenommenen Strahlung proportionale elektrische Ausgangssignale erzeugt und in eine Auswerteschaltung (46) eingegeben werden,
und bei dem der zweiten Seite der Matte (11) eine sich quer zu der Bewegungsrichtung (7) erstreckende Anordnung von Strahlungsquellen (33, 35) vorgesehen wird,
- wobei die Strahlungsquellen (33, 35) quer zu der Bewegungsrichtung (7) der Matte (11) in einem Querabstand (30) voneinander angeordnet werden,
- und wobei die von jeder Strahlungsquelle (33, 35) ausgehende Strahlung zu einem Strahlungsfächer (37) geformt wird,
**dadurch gekennzeichnet,**
- **dass** durch jeden Strahlungsfächer (37) eine Zeile (34, 36) von Detektorelementen (44, 44', 51 ...; 51 ...) der Anordnung von Zeilen (34, 36) von Detektorelementen (44, 44', 51 ... ; 51 ... ) mit der Strahlung beaufschlagt wird,
- **dass** eine Breitenerstreckung (39) jedes Strahlungsfächers (37) quer zu der Bewegungsrichtung (7) der Matte (11) und mit der zugehörigen Zeile (34, 36) von Detektorelementen (44, 44', 51 ... ; 51 ... ) fluchtend angeordnet wird,
- **dass**
- benachbarte Strahlungsfächer (37) und
- die jeweils zugehörigen Zeilen (34, 36) von Detektorelementen (44, 44', 51 ... ; 51 ... )
in der Bewegungsrichtung (7) der Matte (11) in einem Längsabstand (47) voneinander angeordnet werden,
- **dass** distale Enden benachbarter Strahlungsfächer (37) quer zu der Bewegungsrichtung (7) der Matte (11) mit einer Überlappung (40) angeordnet werden,
- und **dass** die Überlappung (40) zumindest über eine Dicke (41) der Matte (11) vorgenommen wird.

2. Verfahren nach Anspruch 1,
**dadurch gekennzeichnet,**
**dass** die durch den fächerförmigen Strahlengang (37) bedingten unterschiedlichen Entfernungen von der Strahlungsquelle (33, 35) bis zur Matte (11), die unterschiedlichen Strahlungswege durch die Matte (11) sowie die unterschiedlich angestrahlten Oberflächen der Detektorelemente (51) trigonometrisch kompensiert werden.

3. Verfahren nach Anspruch 1 oder 2,
**dadurch gekennzeichnet,**
**dass** die durch den fächerförmigen Strahlengang (37) bedingten unterschiedlich orientierten Dichtemessungen der Matte (11) auf der Basis unterschiedlich orientierter Doppelbestimmungen in den Überlappungsbereichen (40) benachbarter Einheiten (31, 32 ; 32, 32) mittels Rechenmodellen in Anlehnung an die an sich bekannte Technik der digitalen Laminographie und Tomosynthese umgerechnet werden.

4. Verfahren nach einem der Ansprüche 1 bis 3,
**dadurch gekennzeichnet,**
- **dass** bei der Prüfung in der Matte (11) angeordnete Fremdkörper (26), wie metallische Gegenstände, Leimklumpen, Kunststoffteile und überdichte Partikelaggregate, festgestellt und entsprechende elektrische Ausgangssignale in die Auswerteschaltung (46) eingegeben werden,
- und **dass** durch die Auswerteschaltung (46) eine Vorrichtung (13) zur Entfernung eines die Fremdkörper (26) enthaltenden Abschnitts der Matte (11) gesteuert wird.

5. Verfahren nach einem der Ansprüche 1 bis 4,
**dadurch gekennzeichnet,**
**dass** bei der Prüfung aus den in die Auswerteschaltung (46) eingegebenen Ausgangssignalen durch die Auswerteschaltung (46) laufend und für die gesamte Fläche der Matte (11) die Masse je Flächeneinheit der Matte (11) in kg/m² ermittelt wird.

6. Verfahren nach einem der Ansprüche 1 bis 5,
**dadurch gekennzeichnet,**
- **dass** an zumindest einem Längsrand (42) der Matte (11) ein äußerer Abschnitt einer äußeren Einheit (31) aus einer äußeren Strahlungsquelle (33) und einer äußeren Zeile (34) der Detektorelemente (44, 44', 51 ...) auf einen Bereich außerhalb des Längsrandes (42) der Matte (11) eingestellt wird,
- **dass** in einem ersten Teil jedes Bereichs außerhalb des Längsrandes (42) der Matte (11) ein Normkörper (43) mit bekannter Masse je Flächeneinheit und wenigstens ein zugehöriges äußeres Detektorelement (44) angeordnet werden,
- **dass** in einem zweiten Teil jedes Bereichs wenigstens ein unmittelbar durch die Strahlung beaufschlagtes äußeres Detektorelement (44') angeordnet wird,
- **dass** mit Hilfe jedes Normkörpers (43) und der Ausgangssignale (45, 45') der äußeren Detektorelemente (44, 44') eine Kalibrierung der zugehörigen äußeren Einheit (31) vorgenommen wird,
- und **dass** wenigstens eine (32) der jeder äußeren Einheit (31) benachbarten, die Matte (11) durchstrahlenden sonstigen Einheiten (32) aus jeweils einer der Strahlungsquellen (35) und einer der sonstigen Zeilen (36) von Detektorelementen (51 ...) mit Hilfe der Ausgangssignale (45, 45') der äußeren Detektorelemente (44, 44') kalibriert wird.

7. Verfahren nach Anspruch 6,
**dadurch gekennzeichnet,**
**dass** alle sonstigen Einheiten (32) mit Hilfe der Ausgangssignale (45, 45') der äußeren Detektorelemente (44, 44') nacheinander oder gleichzeitig kalibriert werden.

8. Verfahren nach Anspruch 6,
**dadurch gekennzeichnet,**
- **dass** zumindest eine erste (32) der sonstigen Einheiten (32) mit Hilfe der Ausgangssignale (45, 45') der äußeren Detektorelemente (44, 44') kalibriert wird,
- **dass** danach eine zweite (32) der sonstigen Einheiten (32) mit Hilfe von Ausgangssignalen der Zeile (36) von Detektorelementen (51) der ersten sonstigen Einheit (32) kalibriert wird,
- und **dass** auf analoge Weise alle übrigen sonstigen Einheiten (32) kalibriert werden.

9. Verfahren zur Prüfung einer sich in einer Richtung (7) bewegenden Matte (11) aus Biomassepartikeln, insbesondere aus Fasern und/oder
Holzspänen, zur Herstellung von Platten (2), nach einem der Ansprüche 1 bis 8,
**dadurch gekennzeichnet,**
- **dass** auf einer ersten Seite der Matte (11) eine sich quer zu der Bewegungsrichtung (7) der Matte (11) erstreckende Anordnung (34, 36) von Detektorelementen (44, 44', 51) vorgesehen wird,
- **dass** die Detektorelemente (51) Strahlung aufnehmen, die von wenigstens einer Strahlungsquelle (33; 35) auf einer der ersten Seite gegenüberliegenden zweiten Seite der Matte (11) ausgegangen ist und die Matte (11) durchdrungen hat,
- **dass** durch jedes Detektorelement (44, 44', 51) der aufgenommenen Strahlung proportionale elektrische Ausgangssignale erzeugt und in eine Auswerteschaltung (46) eingegeben werden,
- **dass** die Ausgangssignale (52) der Detektorelemente (51) über die Breite der Matte (11) zu aufeinanderfolgenden Gruppen von Ausgangssignalen (51) gruppiert werden,
- **dass** die jeweils die Dichte eines Längsstreifens der Matte (11) repräsentierenden Ausgangssignale jeder Gruppe in der Auswerteschaltung (46) zusammengefasst verarbeitet werden,
- und **dass** jede derart verarbeitete Ausgangssignalgruppe als Regelgröße (57) für einen dem betreffenden Längsstreifen zugeordneten Reaktionsmechanismus in einer die Matte (9) aus den Biomassepartikeln streuenden Streumaschine (6) verwendet wird.

10. Verfahren nach Anspruch 9,
**dadurch gekennzeichnet,**
**dass** durch die Auswerteschaltung (46) eine Vorrichtung (13) zur Entfernung eines zu geringe Masse je Flächeneinheit aufweisenden Abschnitts der Matte (11) gesteuert wird.

11. Vorrichtung (12) zur Prüfung einer sich in einer Richtung (7) bewegenden Matte (11) aus Biomassepartikeln, insbesondere aus Fasern und/oder Holzspänen, zur Herstellung von Platten (2),
bei der auf einer ersten Seite der Matte (11) eine sich quer zu der Bewegungsrichtung (7) der Matte (11) erstreckende Anordnung von Zeilen (34, 36) von Detektorelementen (44, 44', 51 ... ; 51 ...) vorgesehen ist,
- wobei die Detektorelemente (44, 44', 51) in einer zu der Matte (11) parallelen Ebene angeordnet werden,
- wobei die Detektorelemente (51) Strahlung aufnehmen, die von einer der ersten Seite gegenüberliegenden zweiten Seite der Matte (11) ausgegangen ist und die Matte (11) durchdrungen hat,
- und wobei durch jedes Detektorelement (44, 44', 51) ein der aufgenommenen Strahlung proportionales elektrisches Ausgangssignal erzeugbar und in eine Auswerteschaltung (46) eingebbar ist,
und bei der auf der zweiten Seite der Matte (11) eine sich quer zu der Bewegungsrichtung erstreckende Anordnung von Strahlungsquellen (33, 35) vorgesehen ist,
- wobei die Strahlungsquellen (33, 35) quer zu der Bewegungsrichtung (7) der Matte (11) in einem Querabstand (30) voneinander angeordnet sind,
- und wobei die von jeder Strahlungsquelle (33, 35) ausgehende Strahlung zu einem Strahlungsfächer (37) geformt ist,
**dadurch gekennzeichnet,**
- **dass** durch jeden Strahlungsfächer (37) eine Zeile (34,36) von Detektorelementen (44, 44', 51 ... ; 51 ...) der Anordnung von Zeilen (34, 36) von Detektorelementen (44, 44', 51 ... ; 51 ...) mit der Strahlung beaufschlagbar ist,
- **dass** eine Breitenerstreckung (39) jedes Strahlungsfächers (37) quer zu der Bewegungsrichtung (7) der Matte (11) und mit der zugehörigen Zeile (34, 36) von Detektorelementen (44, 44', 51 ... ; 51 ...) fluchtend angeordnet ist,
- **dass**
- benachbarte Strahlungsfächer (37) und
- die jeweils zugehörigen Zeilen (34,36) von Detektorelementen (44, 44', 51 ... ; 51 ...)
in der Bewegungsrichtung (7) der Matte (11) in einem Längsabstand (47) voneinander angeordnet sind,
- **dass** distale Enden benachbarter Strahlungsfächer (37) quer zu der Bewegungsrichtung (7) der Matte (11) mit einer Überlappung (40) angeordnet sind,
- und **dass** die Überlappung (40) zumindest über eine Dicke (41) der Matte (11) besteht.

12. Prüfvorrichtung (12) nach Anspruch 11,
**dadurch gekennzeichnet,**
**dass** ein Öffnungswinkel (38) jedes Strahlungsfächers (37) etwa 30° bis 60°, vorzugsweise 44°, beträgt.

13. Prüfvorrichtung (12) nach Anspruch 11 oder 12,
**dadurch gekennzeichnet,**
**dass** alle Strahlungsquellen (33, 35) in gleicher Weise ausgebildet und in einer zu der Matte (11) parallelen Ebene angeordnet sind.

14. Prüfvorrichtung (12) nach einem der Ansprüche 11 bis 13,
**dadurch gekennzeichnet,**
**dass** die Anzahl der Strahlungsquellen (33, 35) wählbar ist.

15. Prüfvorrichtung (12) nach einem der Ansprüche 11 bis 14,
**dadurch gekennzeichnet,**
**dass** der Abstand der Strahlungsquellen (33, 35) voneinander einstellbar ausgebildet ist.

## Claims

1. A method for testing a mat (11), which moves in one direction (7), made of biomass particles, in particular made of fibers and/or wood chips, for producing plates (2),
in which, on a first side of the mat (11), an arrangement of lines (34, 36) of detector elements (44, 44', 51...; 51...), which extends transversely to the movement direction (7) of the mat (11), is provided,
- the detector elements (44, 44', 51) being arranged in a plane parallel to the mat (11),
- the detector elements (51) absorbing radiation which has originated from a second side of the mat (11) opposite to the first side and has penetrated the mat (11),
- and electrical output signals, which are proportional to the absorbed radiation, being generated and input into an analysis circuit (46) by each detector element (44, 44', 51),
and in which, on the second side of the mat (11), an arrangement of radiation sources (33, 35), which extends transversely to the movement direction (7), is provided,
- the radiation sources (33, 35) being arranged in a transverse spacing (30) from one another transversely to the movement direction (7) of the mat (11),
- and the radiation originating from each radiation source (33, 35) being formed into a radiation fan (37),
**characterized in that**
- one line (34, 36) of detector elements (44, 44', 51...; 51...) of the arrangement of lines (34, 36) of detector elements (44, 44', 51...; 51...) is impinged with the radiation by each radiation fan (37),
- a width extension (39) of each radiation fan (37) is arranged transversely to the movement direction (7) of the mat (11) and aligned with the associated line (34, 36) of detector elements (44, 44', 51...; 51...),
- adjacent radiation fans (37) and
the respective associated lines (34, 36) of detector elements (44, 44', 51...; 51...)
are arranged at a longitudinal spacing (47) from one another in the movement direction (7) of the mat (11),
- distal ends of adjacent radiation fans (37) are arranged transversely to the movement direction (7) of the mat (11) with an overlap (40),
- and the overlap (40) occurs at least over one thickness (41) of the mat (11).

2. The method according to Claim 1,
**characterized in that** the different distances from the radiation source (33, 35) up to the mat (11) caused by the fan-shaped beam path (37), the different beam routes through the mat (11), and the different irradiated surfaces of the detector elements (51) are compensated for trigonometrically.

3. The method according to Claim 1 or 2,
**characterized in that** the differently oriented density measurements of the mat (11) caused by the fan-shaped beam path (37) are converted based on differently oriented repeat determinations in the overlap regions (40) of adjacent units (31, 32; 32, 32) by means of computer models in reliance on the technology known per se of digital laminography and tomosynthesis.

4. The method according to one of Claims 1 to 3,
**characterized in that**,
- during the testing, foreign bodies (26) arranged in the mat (11), such as metallic objects, glue lumps, plastic parts, and excessively dense particle aggregates are established and corresponding electrical output signals are input into the analysis circuit (46),
- and a device (13) for removing a section of the mat (11) containing the foreign bodies (26) is controlled by the analysis circuit (46).

5. The method according to one of Claims 1 to 4,
**characterized in that**, during the testing, the mass per unit area of the mat (11) in kg/m² is ascertained by the analysis circuit (46) in a running manner and for the entire area of the mat (11) from the output signals input into the analysis circuit (46).

6. The method according to one of Claims 1 to 5,
**characterized in that**,
- on at least one longitudinal edge (42) of the mat (11), an outer section of an outer unit (31) made of an outer radiation source (33) and an outer line (34) of the detector elements (44, 44', 51...; 51...) is set on a region outside the longitudinal edge (42) of the mat (11),
- in a first part of each region outside the longitudinal edge (42) of the mat (11), a standard body (43) having known mass per unit area and at least one associated outer detector element (44) are arranged,
- in a second part of each region, at least one outer detector element (44') directly impinged by the radiation is arranged,
- with the aid of each standard body (43) and the output signals (45, 45') of the outer detector elements (44, 44'), a calibration of the associated outer unit (31) is performed,
- and at least one (32) of the other units (32), which are adjacent to each outer unit (31) and radiate through the mat (11), each made of one of the radiation sources (35) and one of the other lines (36) of detector elements (51...) is calibrated with the aid of the output signals (45, 45') of the outer detector elements (44, 44').

7. The method according to Claim 6,
**characterized in that** all other units (32) are calibrated successively or simultaneously with the aid of the output signals (45, 45') of the outer detector elements (44, 44').

8. The method according to Claim 6,
**characterized in that**
- at least one first one (32) of the other units (32) is calibrated with the aid of the output signals (45, 45') of the outer detector elements (44, 44'),
- then a second (32) of the other units (32) is calibrated with the aid of output signals of the line (36) of detector elements (51) of the first other unit (32),
- and all remaining other units (32) are calibrated in a similar manner.

9. The method for testing a mat (11), which moves in one direction (7), made of biomass particles, in particular made of fibers and/or wood chips, for producing plates (2), according to one of Claims 1 to 8,
**characterized in that**,
- on a first side of the mat (11), an arrangement (34, 36) of detector elements (44, 44', 51) extending transversely to the movement direction (7) of the mat (11) is provided,
- the detector elements (51) absorb radiation, which has originated from at least one radiation source (33; 35) on a second side of the mat (11) opposite to the first side and has penetrated the mat (11),
- electrical output signals proportional to the absorbed radiation are generated by each detector element (44, 44', 51) and input into an analysis circuit (46),
- the output signals (52) of the detector elements (51) are grouped over the width of the mat (11) into successive groups of output signals (51),
- the respective output signals of each group representing the density of one longitudinal strip of the mat (11) are processed in combination in the analysis circuit (46),
- and each output signal group processed in this manner is used as a control variable (57) for a reaction mechanism assigned to the relevant longitudinal strip in a scattering machine (6) which scatters the mat (9) made of the biomass particles.

10. The method according to Claim 9,
**characterized in that** a device (13) for removing a section of the mat (11) having an excessively low mass per unit area is controlled by the analysis circuit (46).

11. A device (12) for testing a mat (11), which moves in one direction (7), made of biomass particles, in particular made of fibers and/or wood chips, for producing plates (2),
in which, on a first side of the mat (11), an arrangement of lines (34, 36) of detector elements (44, 44', 51...; 51...), which extends transversely to the movement direction (7) of the mat (11), is provided,
- the detector elements (44, 44', 51) being arranged in a plane parallel to the mat (11),
- the detector elements (51) absorbing radiation which has originated from a second side of the mat (11) opposite to the first side and has penetrated the mat (11),
- and an electrical output signal, which is proportional to the absorbed radiation, being able to be generated and input into an analysis circuit (46) by each detector element (44, 44', 51),
and in which, on the second side of the mat (11), an arrangement of radiation sources (33, 35), which extends transversely to the movement direction, is provided,
- the radiation sources (33, 35) being arranged in a transverse spacing (30) from one another transversely to the movement direction (7) of the mat (11),
- and the radiation originating from each radiation source (33, 35) being formed into a radiation fan (37),
**characterized in that**
- one line (34, 36) of detector elements (44, 44', 51...; 51...) of the arrangement of lines (34, 36) of detector elements (44, 44', 51...; 51...) can be impinged with the radiation by each radiation fan (37),
- a width extension (39) of each radiation fan (37) is arranged transversely to the movement direction (7) of the mat (11) and aligned with the associated line (34, 36) of detector elements (44, 44', 51...; 51...),
- adjacent radiation fans (37) and
the respective associated lines (34, 36) of detector elements (44, 44', 51...; 51...)
are arranged at a longitudinal spacing (47) from one another in the movement direction (7) of the mat (11),
- distal ends of adjacent radiation fans (37) are arranged transversely to the movement direction (7) of the mat (11) with an overlap (40),
- and the overlap (40) exists at least over one thickness (41) of the mat (11).

12. The testing device (12) according to Claim 11,
**characterized in that** an aperture angle (38) of each radiation fan (37) is approximately 30° to 60°, preferably 44°.

13. The testing device (12) according to Claim 11 or 12,
**characterized in that** all radiation sources (33, 35) are implemented in a similar manner and are arranged in a plane parallel to the mat (11).

14. The testing device (12) according to one of Claims 11 to 13, **characterized in that** the number of the radiation sources (33, 35) is selectable.

15. The testing device (12) according to one of Claims 11 to 14, **characterized in that** the spacing of the radiation sources (33, 35) from one another is implemented as settable.

## Revendications

1. Procédé pour le contrôle d'une nappe (11) de particules de biomasse, en particulier de fibres et/ou de copeaux de bois, se déplaçant dans une direction (7) pour la fabrication de panneaux (2),
dans lequel est prévu sur une première face de la nappe (11) une disposition de rangées (34, 36) d'éléments détecteurs (44, 44', 51... ; 51...) qui s'étendent perpendiculairement à la direction de déplacement (7) de la nappe (11),
- les elements détecteurs (44, 44', 51) étant disposés dans un plan parallèle à la nappe (11) ;
- les éléments détecteurs (51) captant un rayonnement qui est émis depuis une deuxième face de la nappe (11) opposée à la première face et qui a traversé la nappe (11) ;
- et chaque élément détecteur (44, 44', 51) produisant des signaux électriques proportionnels au rayonnement capté et les transmettant à un circuit d'analyse (46),
et dans lequel il est prévu sur la deuxième face de la nappe (11) une disposition de sources de rayonnement (33, 35) qui s'étend perpendiculairement à la direction de déplacement (7),
- les sources de rayonnement (33, 35) étant disposées perpendiculairement à la direction de déplacement (7) de la nappe (11) à une distance transversale (30) les unes des autres,
- et le rayonnement émis par chaque source de rayonnement (33, 35) étant mis en forme pour donner un éventail de rayons (37),
**caractérisé en ce que**
- chaque éventail de rayons (37) expose au rayonnement und range (34, 36) d'éléments détecteurs (44, 44', 51... ; 51...) de la disposition de rangées (34, 36) d'éléments détecteurs (44, 44', 51... ; 51...),
- **en ce qu'**une étendue en largeur (39) de chaque éventail de rayons (37) est disposée perpendiculairement à la direction de déplacement (7) de la nappe (11) et dans l'alignement de la rangée (34, 36) d'éléments détecteurs (44, 44', 51... ; 51...) correspondante,
- **en ce que**
- des éventail de rayons (37) voisins
et
- les rangées (34, 36) d'éléments détecteurs (44, 44', 51... ; 51...)
correspondantes
sont disposés à une distance longitudinale (47) les uns des autres dans le sens de déplacement (7) de la nappe (11) ;
- **en ce que** les extrémités distales d'éventails de rayons (37) voisins sont disposées perpendiculairement à la direction de déplacement (7) de la nappe (11) avec un chevauchement (40),
- et **en ce que** le chevauchement (40) est réalisé au moins sur une épaisseur (41) de la nappe (11).

2. Procédé selon la revendication 1, **caractérisé en ce que** les différences de distance entre la source de rayonnement (33, 35) et la nappe (11) résultant du trajet des rayons (37) en éventail, les différences de trajet des rayons à travers la nappe (11) et les différences d'incidence du rayonnement sur les surfaces des éléments détecteurs (51) sont compensées de façon trigonométrique.

3. Procédé selon la revendication 1 ou 2, **caractérisé en ce que** les mesures de densité de la nappe (11) orientées différemment en raison du trajet des rayons (37) en éventail sont converties sur la base de doubles déterminations d'orientation différente dans les zones de chevauchement (40) d'unités (31, 32 ; 32, 32) voisines au moyen de modèles de calcul inspirés de la technique connue en soi de laminographie numérique et de tomosynthèse.

4. Procédé selon l'une des revendications 1 à 3, **caractérisé en ce que**
- lors de l'examen, les corps étrangers (26) disposés dans la nappe (11), tels que des objets métalliques, des grumeaux de colle, des morceaux de plastique et des agrégats de particules trop denses, sont repérés et des signaux électriques de sortie correspondants sont transmis au circuit d'analyse (46),
- et **en ce que** le circuit d'analyse (46) commande un dispositif (13) destiné à éliminer la partie de la nappe (11) contenant les corps étrangers (26).

5. Procédé selon l'une des revendications 1 à 4, **caractérisé en ce que** lors de l'examen, la masse par unité de surface de la nappe (11) en kg/m² est déterminée par le circuit d'analyse (46) à partir des signaux de sortie transmis au circuit d'analyse (46) en continu et pour toute la surface de la nappe (11).

6. Procédé selon l'une des revendications 1 à 5, **caractérisé en ce que**
- sur au moins un bord longitudinal (42) de la nappe (11), une section extérieure d'une unité extérieure (31) formée par une source de rayonnement extérieure (33) et une rangée extérieure (34) d'éléments détecteurs (44, 44', 51...) est réglée sur une zone située en dehors du bord longitudinal (42) de la nappe (11),
- **en ce qu'**un étalon (43) ayant une masse par unité de surface connue et au moins un élément détecteur extérieur (44) correspondant sont disposés dans chaque partie de chaque zone en dehors du bord longitudinal (42) de la nappe (11),
- **en ce qu'**au moins un élément détecteur extérieur (44') directement exposé au rayonnement est disposé dans une deuxième partie de chaque zone,
- **en ce qu'**à l'aide de chaque étalon (43) et des signaux de sortie (45, 45') des éléments détecteurs extérieurs (44, 44'), l'unité extérieure (31) correspondante est étalonnée,
- et **en ce qu'**au moins une (32) des autres unités (32) voisines de chaque unité extérieure (31) dont le rayonnement traverse la nappe (11), composée de l'une des sources de rayonnement (35) et de l'une des autres rangées (36) d'éléments détecteurs (51...), est étalonnée à l'aide des signaux de sortie (45, 45') des éléments détecteurs extérieurs (44, 44').

7. Procédé selon la revendication 6, **caractérisé en ce que** toutes les autres unités (32) sont étalonnées successivement ou simultanément à l'aide des signaux de sortie (45, 45') des éléments détecteurs extérieurs (44, 44').

8. Procédé selon la revendication 6, **caractérisé en ce**
- **qu'**au moins une première (32) des autres unités (32) est étalonnée à l'aide des signaux de sortie (45, 45') des éléments détecteurs extérieurs (44, 44'),
- **qu'**une deuxième (32) des autres unités (32) est ensuite étalonnée à l'aide des signaux de sortie de la rangée (36) d'éléments détecteurs (51) de la première des autres unités (32),
- et **que** toutes les autres unités (32) restantes sont étalonnées de manière analogue.

9. Procédé pour le contrôle d'une nappe (11) de particules de biomasse, en particulier de fibres et/ou de copeaux de bois, se déplaçant dans une direction (7) pour la fabrication de panneaux (2), selon l'une des revendications 1 à 8,
**caractérisé en ce que**
- il est prévu sur une première face de la nappe (11) une disposition (34, 36) d'éléments détecteurs (44, 44', 51) s'étendant perpendiculairement à la direction de déplacement (7) de la nappe (11),
- **en ce que** les éléments détecteurs (51) captent un rayonnement qui est parti d'au moins une source de rayonnement (33 ; 35) sur une deuxième face de la nappe (11) opposée à la première face et qui a traversé la nappe (11),
- **en ce que** chaque élément détecteur (44, 44', 51) produit des signaux électriques proportionnels au rayonnement capté et les transmet à un circuit d'analyse (46),
- **en ce que** les signaux de sortie (52) des éléments détecteurs (51) sont regroupés sur la largeur de la nappe (11) en groupes successifs de signaux de sortie (51),
- **en ce que** les signaux de sortie de chaque groupe représentant chacun la densité d'une bande longitudinale de la nappe (11) sont traités ensemble dans le circuit d'analyse (46),
- et **en ce que** chaque groupe de signaux de sortie ainsi traité est utilisé comme grandeur de régulation (57) pour un mécanisme de réaction associé à la bande longitudinale en question dans une épandeuse (6) qui répand la nappe (9) à partir des particules de biomasse.

10. Procédé selon la revendication 9, **caractérisé en ce que** le circuit d'analyse (46) commande un dispositif (13) pour éliminer une section de la nappe (11) présentant une masse par unité de surface trop faible.

11. Dispositif (12) pour le contrôle d'une nappe (11) de particules de biomasse, en particulier de fibres et/ou de copeaux de bois, se déplaçant dans une direction (7) pour la fabrication de panneaux (2),
dans lequel est prévue sur une première face de la nappe (11) une disposition de rangées (34, 36) d'éléments détecteurs (44, 44', 51... ; 51...) qui s'étend perpendiculairement à la direction de déplacement (7) de la nappe (11),
- les éléments détecteurs (44, 44', 51) étant disposés dans un plan parallèle à de la nappe (11),
- les éléments détecteurs (51) absorbant un rayonnement qui est parti d'une deuxième face de la nappe (11) opposée à la première face et qui a traversé la nappe (11),
- et chaque élément détecteur (44, 44', 51) pouvant produire un signal de sortie électrique proportionnel au rayonnement capté et pouvant le transmettre à un circuit d'analyse (46),
et dans lequel est prévue sur la deuxième face de la nappe (11) une disposition de sources de rayonnement (33, 35) qui s'étend perpendiculairement à la direction de déplacement,
- les sources de rayonnement (33, 35) étant disposées perpendiculairement à la direction de déplacement (7) de la nappe (11) à une distance transversale (30) les unes des autres,
- et le rayonnement émis par chaque source de rayonnement (33, 35) étant mis en forme pour donner un éventail de rayons (37),
**caractérisé en ce que**
- chaque éventail de rayons (37) peut exposer au rayonnement une rangée (34, 36) d'éléments détecteurs (44, 44', 51... ; 51...) de la disposition de rangées (34, 36) d'éléments détecteurs (44, 44', 51... ; 51...),
- **en ce qu'**une étendue en largeur (39) de chaque éventail de rayons (37) est disposée perpendiculairement à la direction de déplacement (7) de la nappe (11) et dans l'alignement de la rangée (34, 36) d'éléments détecteurs (44, 44', 51... ; 51...) correspondante,
- **en ce que**
- des éventail de rayons (37) voisins
et
- les rangées (34, 36) d'éléments détecteurs (44, 44', 51... ; 51...) correspondantes
sont disposés à une distance longitudinale (47) les uns des autres dans le sens de déplacement (7) de la nappe (11) ;
- **en ce que** les extrémités distales d'éventails éventail de rayons (37) voisins sont disposées perpendiculairement à la direction de déplacement (7) de la nappe (11) avec un chevauchement (40),
- et **en ce que** le chevauchement (40) est réalisé au moins sur une épaisseur (41) de la nappe (11).

12. Dispositif de contrôle (12) selon la revendication 11, **caractérisé en ce qu'**un angle d'ouverture (38) de chaque éventail de rayons (37) est d'environ 30° à 60°, de préférence de 44°.

13. Dispositif de contrôle (12) selon la revendication 11 ou 12, **caractérisé en ce que** toutes les sources de rayonnement (33, 35) sont construites de la même manière et sont disposées dans un plan parallèle à la nappe (11).

14. Dispositif de contrôle (12) selon l'une des revendications 11 à 13, **caractérisé en ce que** le nombre de sources de rayonnement (33, 35) peut être choisi.

15. Dispositif de contrôle (12) selon l'une des revendications 11 à 14, **caractérisé en ce que** la distance entre les sources de rayonnement (33, 35) est réglable.
